(19) Europäisches Patentamt
European Patent Office
Office européen des brevets

(11) **EP 3 199 202 B1**

(12) **EUROPEAN PATENT SPECIFICATION**

(45) Date of publication and mention
of the grant of the patent:
**16.09.2020 Bulletin 2020/38**

(51) Int Cl.:
***A61N 5/10*** *(2006.01)*

(21) Application number: **17153697.2**

(22) Date of filing: **30.01.2017**

(54) **TREATMENT PLANNING DEVICE, TREATMENT PLANNING METHOD, CONTROL DEVICE, AND PARTICLE BEAM TREATMENT SYSTEM**

BEHANDLUNGSPLANUNGSVORRICHTUNG, BEHANDLUNGSPLANUNGSVERFAHREN, STEUERUNGSVORRICHTUNG UND PARTIKELSTRAHLBEHANDLUNGSSYSTEM

DISPOSITIF DE PLANIFICATION DE TRAITEMENT, PROCÉDÉ DE PLANIFICATION DE TRAITEMENT, DISPOSITIF DE COMMANDE ET SYSTÈME DE TRAITEMENT À FAISCEAU DE PARTICULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2016 JP 2016013903**

(43) Date of publication of application:
**02.08.2017 Bulletin 2017/31**

(73) Proprietor: **Hitachi, Ltd.**
**Chiyoda-ku,**
**Tokyo 100-8280 (JP)**

(72) Inventors:
• **FUJITAKA, Shinichiro**
**Tokyo, 100-8280 (JP)**

• **HIRAYAMA, Shusuke**
**Tokyo, 100-8280 (JP)**
• **UMEZAWA, Masumi**
**Tokyo, 100-8280 (JP)**

(74) Representative: **MERH-IP Matias Erny Reichl Hoffmann**
**Patentanwälte PartG mbB**
**Paul-Heyse-Strasse 29**
**80336 München (DE)**

(56) References cited:
| | |
|---|---|
| **EP-A1- 2 305 351** | **EP-A1- 2 392 383** |
| **EP-A1- 2 923 731** | **WO-A1-2014/072213** |
| **US-A1- 2004 104 354** | **US-A1- 2015 080 631** |

**Description**

Technical Field

The present invention relates to a particle beam treatment device which treats a cancer by irradiating an affected area with a charged particle beam accelerated by a synchrotron or cyclotron particle beam accelerator.

Background Art

As a method of irradiating an affected area with a charged particle beam in particle beam treatment, a scanning irradiation method is known in which an irradiation target is irradiated with a charged particle while being directly scanned. In particle beam scanning irradiation, in order to irradiate the affected area with a uniform irradiation dose, a plurality of scanning methods are used. In scanning irradiation method called dispersed spot irradiation, irradiation spots to be irradiated with a beam are arranged in the affected area, and a treatment planning device determines a target irradiation dose for each irradiation spot. When the affected area is irradiated, an irradiation position and an irradiation dose of the charged particle beam are measured, and the determined irradiation spots are irradiated with the beam as much as a predetermined irradiation dose. If one irradiation spot is completely irradiated with the beam, a beam is stopped (turned off) once, and is moved to the subsequent irradiation spot. The beam is turned on again so as to irradiate the subsequent irradiation spot. This procedure is repeatedly performed on all of the irradiation spots, thereby completing the irradiation. In order to change energy in a depth direction of the affected area, energy of the charged particle beam is changed by an accelerator, thereby changing the irradiation spot in the depth direction.

In addition, as another scanning method, an irradiation method called continuous beam irradiation is known. This method is the same as the dispersed spot irradiation in that the beam moves to the subsequent irradiation spot if the irradiation spot is completely irradiated as much as the irradiation dose. However, according to the irradiation method, even when beam moves between the irradiation spots, the beam moves while irradiating the irradiation spot with the beam. In order to perform scanning irradiation using the continuous beam, it is necessary not only to consider the irradiation dose for irradiating a portion between the irradiation spots with the charged particle beam, but also to cause a treatment planning device to calculate an irradiation plan which enables the affected area to be irradiated with a uniform irradiation dose. PTL 1 discloses the following method. In a case where the continuous beam irradiation is performed, when the irradiation spots are separated from each other, the irradiation spots are classified into groups, thereby determining a scanning route.

Other scanning methods are disclosed in PTL 2, PTL 3 and PTL 4. PTL 2 discloses methods applying beam rescanning in order to deliver a target dose to each spot.

Citation List

Patent Literature

PTL 1: Japanese Patent No. 5791793
PTL 2: EP 2392383 A1
PTL 3: US 2015/0080631 A1
PTL 4: WO 2014/072213

Summary of Invention

Technical Problem

According to a technique disclosed in PTL 1, when the irradiation spots are separated from each other, the irradiation has to be performed by lowering beam intensity so that the irradiation dose for the portion between the irradiation spots does not increase. In addition, in a case where the irradiation spots which have mutually different magnitudes of the target irradiation dose are mixed with each other, it is necessary to perform the irradiation in accordance with the irradiation spot whose target irradiation dose is small. For these reasons, in a case where the continuous beam irradiation is performed, the irradiation cannot be performed by raising the beam intensity. Consequently, a treatment time tends to be lengthened.

Solution to Problem

In order to solve the above-described problem, the present invention adopts configurations described in Claims.

The present application includes a plurality of means for solving the above-described problem. As an example, there is provided a treatment planning device including a spot determination unit that classifies an irradiation region to be irradiated with a charged particle beam into a plurality of layers in an irradiation direction of the charged particle beam, and that arranges a plurality of irradiation spots which serve as irradiation positions of the charged particle beam, in the layers, a group classification unit that classifies the irradiation spots into groups in accordance with at least either a distance between one irradiation spot and another irradiation spot which are arranged in the same layer or a target irradiation dose of each irradiation spot, and/or a planning unit that prepares a plan so as to continuously emit the charged particle beam while the irradiation position is changed from an irradiation spot to a subsequent irradiation spot belonging to a certain group, and so as to stop emitting the charged particle beam while the irradiation position is changed from an irradiation spot belonging to a certain group to another irradiation spot belonging to another group located in the same layer as that of the certain group.

Advantageous Effects of Invention

According to the present invention, a treatment time can be shortened in particle beam scanning irradiation.

Brief Description of Drawings

[Fig. 1] Fig. 1 is a view illustrating an overall configuration of a particle beam treatment system.
[Fig. 2] Fig. 2 is a view illustrating a particle beam scanning irradiation nozzle.
[Fig. 3] Fig. 3 is a view illustrating a layer irradiated with the same energy, a charged particle beam, and irradiation spots, when scanning irradiation is performed on an affected area.
[Fig. 4] Fig. 4 is a view illustrating irradiation dose distribution in a depth direction when the scanning irradiation is performed on the affected area.
[Fig. 5] Fig. 5 is a view illustrating continuous beam irradiation.
[Fig. 6] Fig. 6 is a view illustrating dispersed spot irradiation.
[Fig. 7] Fig. 7 is a view illustrating control for the continuous beam irradiation.
[Fig. 8] Fig. 8 is a view illustrating control for the dispersed spot irradiation.
[Fig. 9] Fig. 9 is a flowchart illustrating an exemplary embodiment.
[Fig. 10] Fig. 10 is a view illustrating an arrangement of the irradiation spots.
[Fig. 11] Fig. 11 is a view illustrating group classification in accordance with a distance relationship between the irradiation spots.
[Fig. 12] Fig. 12 is a view illustrating scanning irradiation in which continuous beam irradiation and dispersed spot irradiation are combined with each other
[Fig. 13] Fig. 13 is a view for describing moving between the irradiation spots and stopping at the irradiation spot when the continuous beam irradiation is performed.
[Fig. 14] Fig. 14 is a view illustrating continuous beam irradiation in the related art.
[Fig. 15] Fig. 15 is a flowchart illustrating the : exemplary embodiment.
[Fig. 16] Fig. 16 is a flowchart illustrating a second exemplary embodiment.
[Fig. 17] Fig. 17 is a flowchart illustrating a third exemplary embodiment.

Description of Embodiments

Specific embodiments will be described in detail with reference to the drawings.

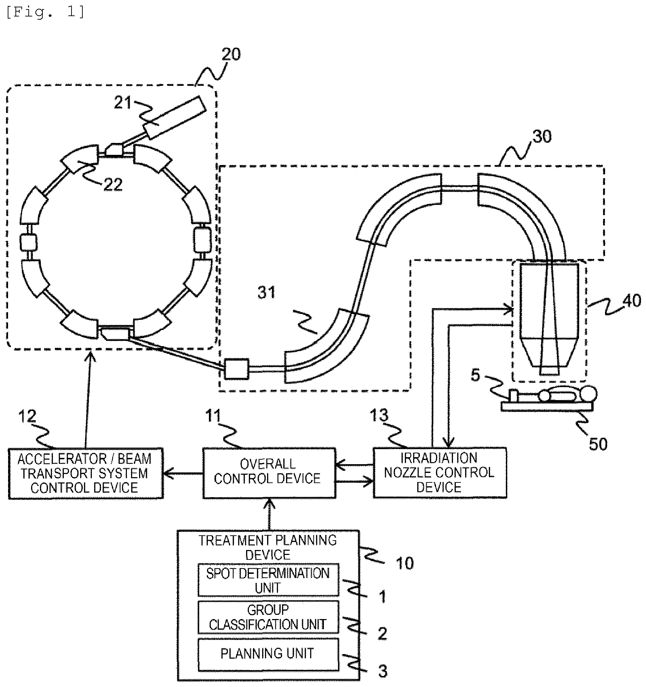

Fig. 1 illustrates an overall configuration of a particle beam treatment system. The particle beam treatment system includes an accelerator 20 that accelerates a charged particle beam (hereinafter, referred to as a beam) 90, a beam transport system 30 that transports the accelerated beam 90 to an irradiation nozzle, an irradiation nozzle 40 that irradiates an affected area with the beam, a treatment table 50, a treatment planning device 10 that prepares a treatment plan, an overall control device 11, an accelerator / beam transport system control device 12, and an irradiation nozzle control device 13. Here, the treatment planning device 10 includes a spot determination unit 1, a group classification unit 2, and a planning unit 3. In addition, the accelerator 20 includes a beam emitter 21 and a synchrotron accelerator 22. The beam 90 accelerated up to 60% to 70% of light speed by the accelerator 20 is transported to the irradiation nozzle 40 while being deflected in vacuum in a magnetic field by a deflection electromagnet 31 disposed in the beam transport system 30. The beam 90 is shaped in the irradiation nozzle 40 so as to match a shape of an irradiation region, and an irradiation target is irradiated with the beam 90. For example, the irradiation target is an affected area 51 of a patient 5 lying on the treatment table 50.

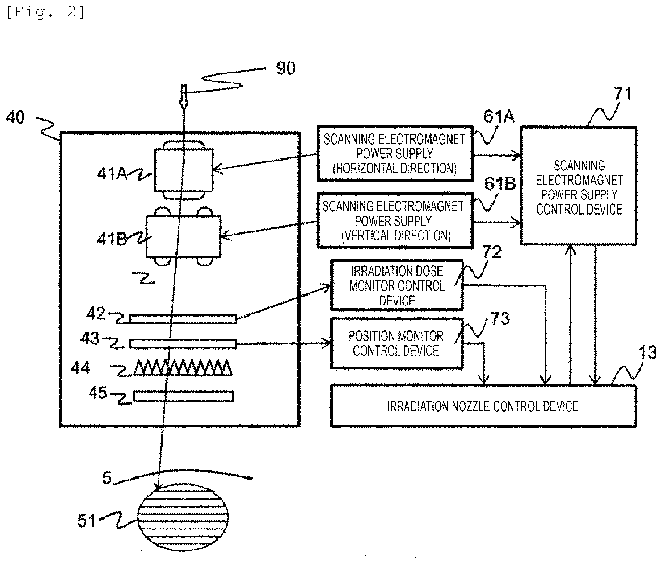

Fig. 2 illustrates the irradiation nozzle 40 for particle beam scanning. In the irradiation nozzle 40, scanning electromagnets 41A and 41B for horizontal and vertical directions scan the inside of a two-dimensional plane with the beam 90. The affected area 51 is irradiated with the beam 90 used in the scanning performed by the scanning electromagnets 41A and 41B. An irradiation dose monitor 42 measures an irradiation dose of the beam 90 used in irradiating each irradiation spot. An irradiation dose monitor control device 72 controls the irradiation dose for irradiating each irradiation spot. A position monitor 43 measures a beam position (for example, a position of the center of gravity) of each irradiation spot. A position monitor control device 73 calculates a position and a width of the irradiation spot, based on data of the beam position measured by the position monitor 43, and confirms an irradiation position of the beam 90. When necessary, a ridge filter 44 is used in order to thicken a Bragg peak. In addition, an arrival position of the beam 90 may be adjusted by inserting a range shifter 45.

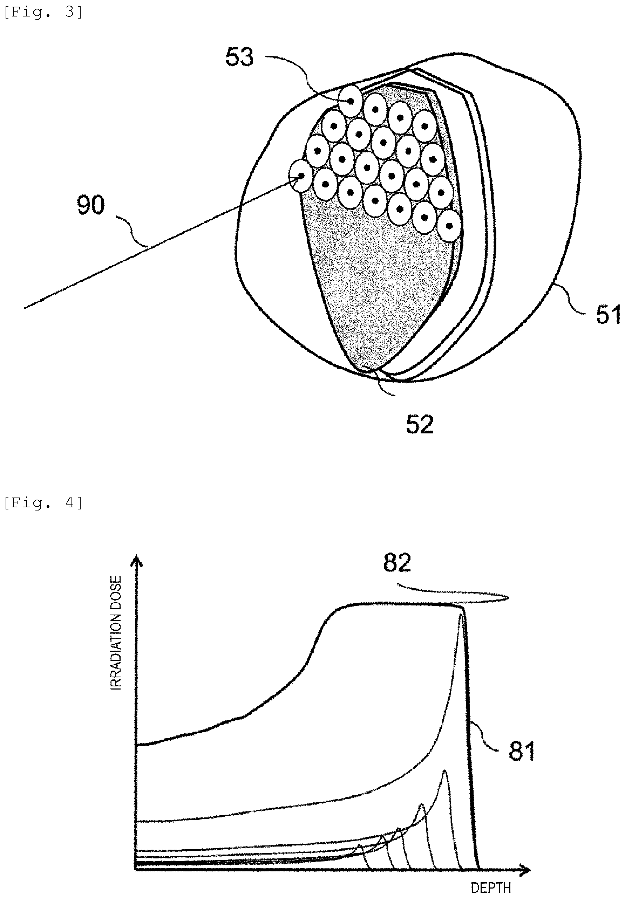

When the scanning irradiation is performed, the treatment planning device 10 illustrated in Fig. 1 calculates positions of the irradiation spots and a target irradiation dose for each irradiation spot in advance in order to irradiate the affected area with a uniform irradiation dose. Fig. 3 illustrates particle beam scanning irradiation. The affected area 51 is classified into layers 52, and the inside of each layer 52 is irradiated with the beam 90 having the same energy. Irradiation spots 53 are arranged inside the layer 52.

Data for each patient which is calculated by the treatment planning device 10 illustrated in Fig. 1 is sent to the overall control device 11 of the particle beam treatment system illustrated in Fig. 1. An energy changing signal, a beam emission start signal, or a beam emission stop signal is output from the overall control device 11 to the accelerator / beam transport system control device 12. A coordinate value and an irradiation dose of each irradiation spot are sent from the overall control device 11 to the irradiation nozzle control device 13. The coordinate value of the irradiation spot is converted into an excitation current value of the scanning electromagnets 41A and 41B. The excitation current value is sent to a scanning electromagnet power supply control device 71 illustrated in Fig. 2.

If a certain irradiation spot 53 arranged by the treatment planning device is irradiated with the beam 90 of a determined irradiation dose, a subsequent irradiation spot 53 is irradiated. If a certain layer 52 is completely irradiated, the subsequent layer 52 is irradiated. First, beam energy is changed in order to change the irradiation position in a beam irradiation direction, that is, in a depth direction of the affected area. If the beam energy is changed, a position of the beam arriving at the inside a body is changed. The charged particle beam having high energy arrives at a deep position inside the body, and the charged particle beam having low energy arrives at only a shallow position inside the body. When the particle beam scanning irradiation is performed, the beam energy is changed in order to form uniform irradiation dose distribution in the depth direction, and the irradiation dose is properly distributed, thereby forming a spread out Bragg peak (SOBP) in the depth direction. Each energy irradiation dose is properly distributed. In this manner, energy Bragg curves 81 are superimposed together, thereby forming uniform irradiation dose distribution SOBP82 in the depth direction as illustrated in Fig. 4.

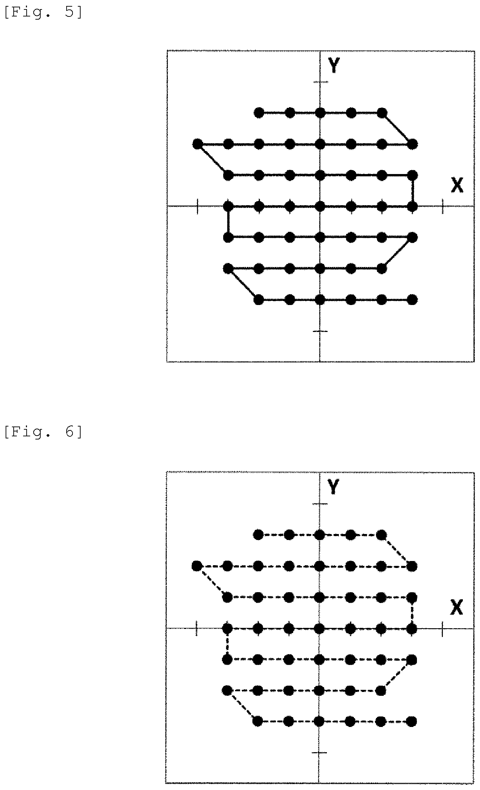

Next, irradiation in the horizontal direction for the scanning irradiation will be described. In the treatment planning device 10, the irradiation spots for irradiating the affected area with a uniform irradiation dose are arranged for each energy of the beam as illustrated in Fig. 3. Fig. 5 illustrates the scanning irradiation using continuous beam irradiation. Fig. 6 illustrates the scanning irradiation using dispersed spot irradiation. A black point represents the irradiation spot. A solid line represents that a beam moves between the irradiation spots while being turned on. A dotted line represents that the beam moves between the irradiation spots while being turned off. As illustrated in Fig. 5, in a case of the continuous beam irradiation, if an irradiation spot is irradiated with the beam as much as the irradiation dose of each irradiation spot which is determined by a treatment plan, the beam moves to the subsequent irradiation spot without turning off the beam. Therefore, the irradiation dose for the irradiation spots is a sum of the irradiation dose used in the irradiation while the beam moves between the irradiation spots and the irradiation dose used in the irradiation while the beam stops at the irradiation spots. The treatment plan corresponding to the continuous beam irradiation determines in advance a scanning route for scanning the irradiation spots as illustrated by the solid line in Fig. 5. In a case of the dispersed spot irradiation illustrated in Fig. 6, the same scanning route as that of the continuous beam irradiation in Fig. 5 is illustrated. However, the beam moves to the subsequent irradiation spot while being turned off when the beam moves between the irradiation spots. Therefore, the irradiation dose is provided for only the spots illustrated by the black point in Fig. 6.

In the continuous beam irradiation, the beam moves between the irradiation spots while the beam is also turned on between the irradiation spots. Accordingly, it is necessary to prepare an irradiation plan for irradiating the affected area with a uniform irradiation dose also in view of the irradiation dose given by the irradiation while the beam moves between the spots . For example, a method may be employed which optimizes the irradiation dose by arranging virtual irradiation spots between the irradiation spots and causing the virtual spots to represent the irradiation dose during the movement. In addition, in the continuous beam irradiation, in order to consider the irradiation dose given by the beam in the irradiation while the beam moves between the irradiation spots, it is necessary to determine a scanning route for scanning the irradiation spots inside a layer to be irradiated with the same energy. For example, a method may be employed which determines the scanning route so as to minimize the scanning distance for scanning the irradiation spots by using a traveling salesman algorithm.

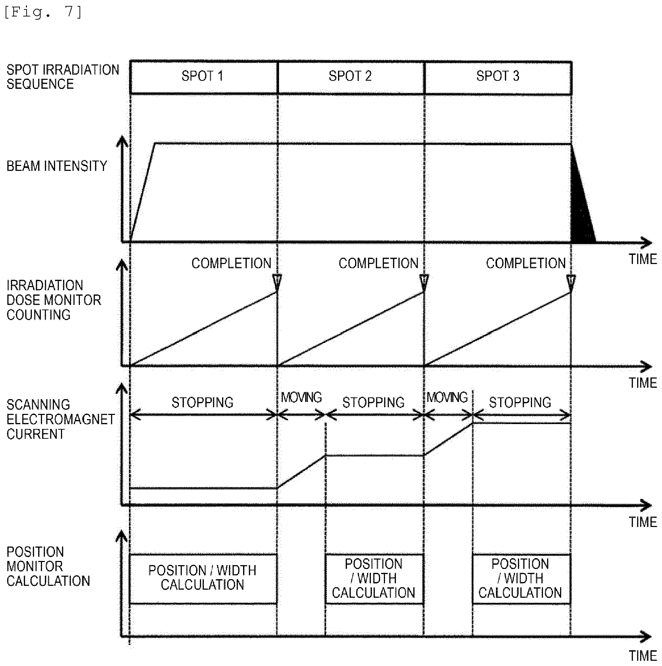

Controls for the continuous beam irradiation and the dispersed spot irradiation will be described in detail. Fig. 7 illustrates a time chart of the continuous beam irradiation. As an example, Fig. 7 illustrates irradiation at three spots from a spot 1 to a spot 3. The accelerator / beam transport system control device 12 illustrated in Fig. 1 instructs the accelerator 20 to irradiate the spots with predetermined beam intensity. If the beam irradiation starts, an ionization output of the irradiation dose monitor 42 inside the irradiation nozzle 40 is converted into pulses by the irradiation dose monitor control device 72, and a pulse count value starts to increase. If a predetermined irradiation dose is achieved, the irradiation dose monitor control device 72 sends a completion signal to the irradiation nozzle control device 13, and the irradiation of the spot is completed. In accordance with the completion signal of the irradiation dose monitor control device 72, the irradiation nozzle control device 13 sends to the scanning electromagnet power supply control device 71 a signal to move to the subsequent spot, and the irradiation nozzle 40 starts to move to the subsequent spot. If a current value of the subsequent spot is attained, the scanning electromagnet power supply control device 71 sends a movement completion signal to the irradiation nozzle control device 13. After receiving a stop completion signal, the position monitor control device 73 obtains an output from the position monitor 43, and starts to calculate a position and a width of the beam. In accordance with an irradiation dose completion signal, the position monitor control device 73 completes the calculation of the position and the width of the beam. The irradiation nozzle control device 13 determines whether or not a predetermined position is irradiated. As a result of the determination, when the position and the width of the beam are greatly deviated, the beam irradiation is stopped. Hitherto, a flow in the control for the continuous beam irradiation has been described.

At the final spot of the continuous beam irradiation, the accelerator 20 has a limited response time. Thus, in some cases, a delayed charge may be generated after the emission stop signal of the beam is output. Predictive control is performed on the delayed charge, and the beam is turned off beforehand. In this manner, it is possible to control the continuous beam irradiation so as to provide the predetermined irradiation dose.

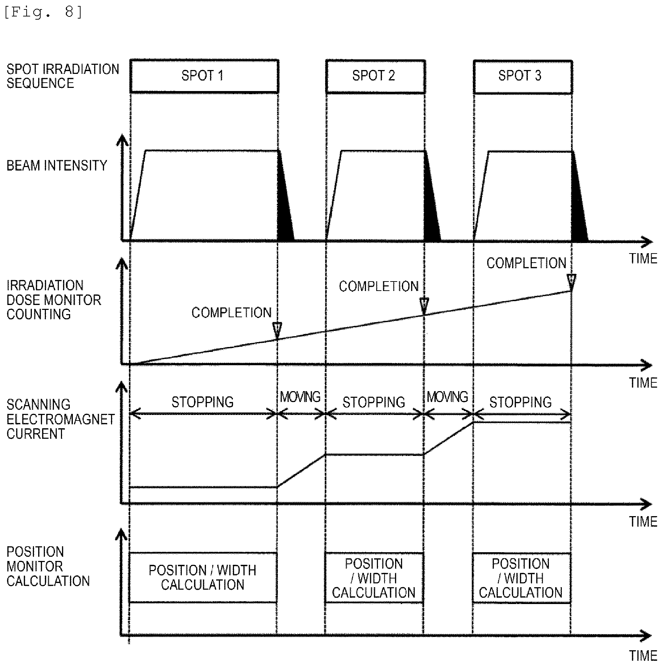

Next, Fig. 8 illustrates a time chart of the dispersed spot irradiation. The dispersed spot irradiation is the same as the continuous beam irradiation in that the beam moves to the subsequent spot in response to the irradiation dose completion signal by changing the current of the scanning electromagnet. In the dispersed spot irradiation, after receiving the irradiation dose completion signal from the irradiation dose monitor control device 72, the irradiation nozzle control device 13 sends an instruction to turn off the beam to the accelerator / beam transport system control device 12 via the overall control device 11, thereby turning off the beam. Thereafter, after the turn-off of the beam is completed, the accelerator / beam transport system control device 12 sends a movement start signal to the irradiation nozzle control device 13 via the overall control device 11. The irradiation nozzle control device 13 receives the movement start signal, and sends a signal to move to the subsequent spot to the scanning electromagnet power supply control device 71. After receiving a movement completion signal of the scanning electromagnet power supplies 61A and 61B, the irradiation nozzle control device 13 sends an instruction to turn on the beam to the accelerator / beam transport system control device 12. In this manner, the beam irradiation starts again, and the subsequent spot starts to be irradiated. The calculation of the position and the width of the beam starts after the movement completion signal is received. The calculation is completed after the completion signal of the irradiation spot is received. Since the beam is turned off between the respective spots, a delay component is present in the irradiation dose compared to the response time of the accelerator 20. This causes the irradiation dose at each spot to increase as much as the amount of the delay component. Therefore, in the dispersed spot irradiation, all of the irradiation doses are integrated and managed, thereby ensuring accuracy in the irradiation dose.

Hitherto, the controls for the continuous beam irradiation and the dispersed spot irradiation have been described. In the continuous beam irradiation, even while the beam moves between the irradiation spots, the beam irradiation is performed. In contrast, in the dispersed spot irradiation, while the beam moves between the irradiation spots, the beam irradiation is not performed. Accordingly, the continuous beam irradiation can shorten a treatment time compared to the dispersed spot irradiation. On the other hand, since the dispersed spot irradiation does not have the irradiation dose for irradiating a portion between the spots, each spot can be very accurately irradiated.

If irradiation can be performed in both the continuous beam irradiation and the dispersed spot irradiation according to the above-described configurations, it is desirable to perform both of these from a viewpoint of the treatment time and accurate irradiation. Since the control device corresponding to two irradiation methods of the dispersed spot irradiation and the continuous beam irradiation is provided, more flexible scanning irradiation can be realized. Here, the same irradiation nozzle may be configured so that both the dispersed spot irradiation and the continuous beam irradiation can be performed. Since the irradiations can be performed using the same irradiation nozzle, it is possible to shorten a time needed to switch between the dispersed spot irradiation and the continuous beam irradiation.

[Embodiment 1]

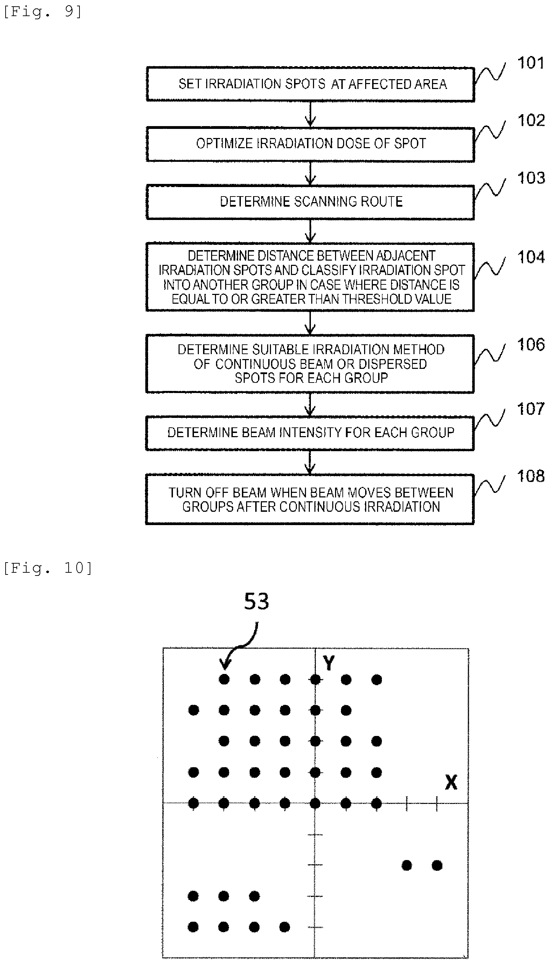

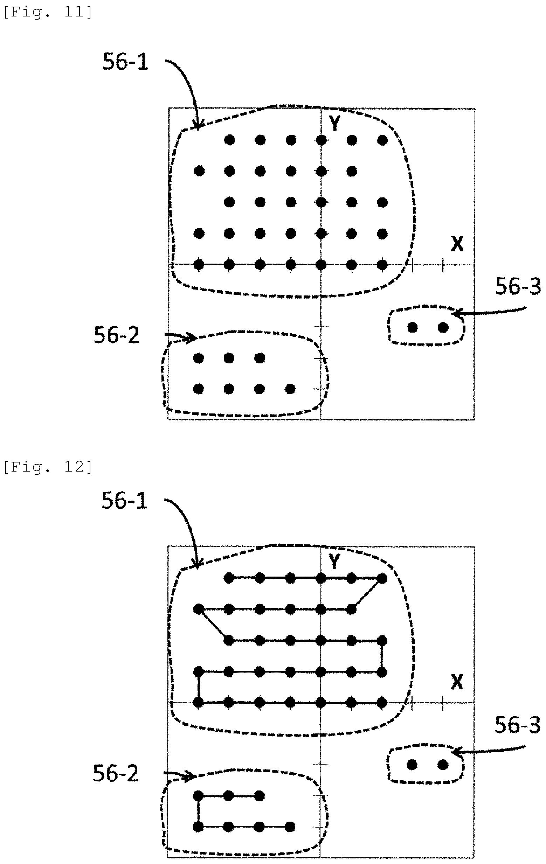

A first exemplary embodiment not forming the invention will be described with reference to the drawings. Fig. 9 illustrates a flowchart according to the present embodiment. When the treatment planning device calculates an irradiation plan for the continuous beam irradiation, the spot determination unit 1 included in the treatment planning device first classifies the affected area 51 into the layers 52, and sets the irradiation spots 53 as illustrated in Fig. 10 (Step 101) . Next, through repeated calculation using the quasi-Newton method, the irradiation dose is optimized so that the irradiation dose for the affected area reached a predetermined irradiation dose (Step 102). As a result, a target irradiation dose for each irradiation spot is determined. Next, a scanning route inside the layer is determined (Step 103). The traveling salesman algorithm or the like is used in determining the scanning route, thereby reducing the total scanning distance. After the scanning route is determined, the group classification unit 2 calculates a distance between the adjacent spots, and performs processing on the spots which are separated from each other with a certain distance or farther so as to be classified into another group (Step 104). Through the processes in Steps 103 and 104, the irradiation spots inside the layer 52 are classified into a plurality of groups. Here, a group includes one or more irradiation spots. For example, group classification is performed as illustrated in Fig. 11.

If the irradiation spots inside the layer 52 are classified into the plurality of groups, the planning unit 3 determines whether to perform the continuous beam irradiation or the dispersed spot irradiation on each group (Step 106). In groups 56-1 and 56-2 in Fig. 11, many spots are densely present. Accordingly, the continuous beam irradiation is performed. In a group 56-3 in Fig. 11, only two spots are present. Accordingly, the dispersed spot irradiation is performed. In the continuous beam irradiation, the irradiation position is changed in a state where the beam is continuously emitted. Therefore, compared to a case where the dispersed spot irradiation is performed on all of the irradiation spots, it is possible to shorten the treatment time by selecting a proper irradiation method.

If the irradiation method is determined as illustrated in Fig. 12, with regard to the group on which the continuous beam irradiation is performed, beam intensity is determined for each group (Step 107). In this case, the beam intensity may be changed for each group.

In accordance with the above-described flow illustrated in Fig. 9, the treatment planning device 10 determines the scanning route and the irradiation method for irradiating the irradiation spots inside the layer. When necessary, in view of the irradiation dose during the movement, the irradiation dose is optimized again.

A method of determining the beam intensity in the continuous beam irradiation in Step 107 will be described. In the continuous beam irradiation, even while the beam moves between the irradiation spots, the beam irradiation is continuously performed. Accordingly, it is necessary to manage the irradiation dose while the beam moves between the irradiation spots. In the continuous beam irradiation, a moving time between the irradiation spots is determined by the distances and the scanning speed between the irradiation spots . Accordingly, the irradiation dose for irradiating the portion between the irradiation spots is managed by controlling the beam intensity of the beam emitted from the accelerator 20 so as to be constant. The distance between the irradiation spots is determined by the treatment plan, based on a shape of the affected area. In addition, the scanning speed is determined if the beam energy is determined by the design of the scanning electromagnets 41A and 41B arranged in the irradiation nozzle 40.

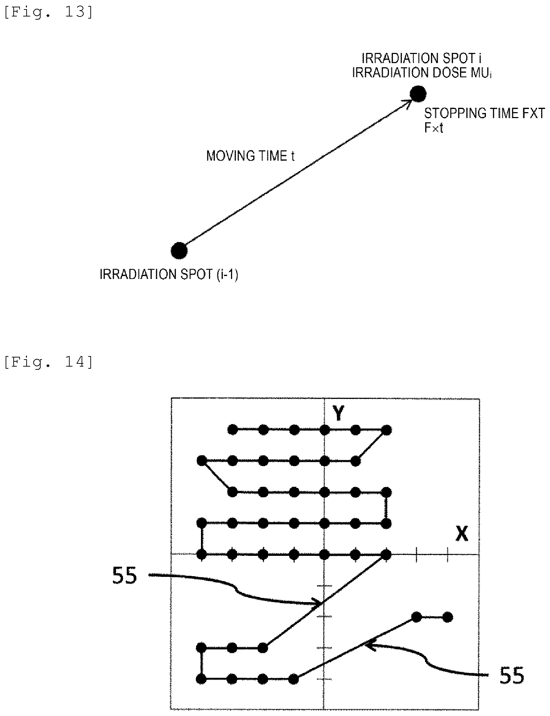

Fig. 13 illustrates two adjacent irradiation spots, and the solid line represents the scanning route through which the beam moves while being turned on. In the continuous beam irradiation, in view of a ratio of the movement of the beam between the irradiation spots and stopping at the irradiation spots, the beam intensity for irradiating each layer is determined. First, the moving time of the beam between the irradiation spots can be calculated in such a way that the interval between the irradiation spots is divided by the scanning speed. The scanning speed of the irradiation nozzle varies between the X-direction and the Y-direction. If the scanning speeds in the X-direction and the Y-direction are respectively set to Vx and Vy and the spot intervals in the X-direction and the Y-direction are respectively set to Lx and Ly, moving times tx and ty in the X-direction and the Y-direction are respectively calculated as follows.
[Expression 1]

$$t_X = \frac{L_X}{V_X} \qquad t_Y = \frac{L_Y}{V_Y} \qquad\qquad (1)$$

A moving time t between the adjacent irradiation spots illustrated in Fig. 12 is maximized in the moving times in the X-direction and the Y-direction, and is expressed as follows.
[Expression 2]

$$t = \max(t_X, t_Y) \tag{2}$$

Here, if the ratio between the stopping and the movement is set to F (value obtained in such a way that the stopping time is divided by the moving time) and the irradiation dose for the subsequent i-th number of irradiation spot is set to MUi, the beam has constant intensity, and the irradiation spot is irradiated with the irradiation dose MUi during a period of the moving time t and the stopping time Ft. Accordingly, beam intensity Ii of the beam for irradiating the i-th number of irradiation spot is obtained as follows.

[Expression 3]

$$I_i = \frac{MU_i}{(1 + F)t} \tag{3}$$

This is repeatedly calculated for all of the irradiation spots on a layer irradiated with the same energy so as to select the minimum current. In this manner, beam intensity I for the layer is determined.

[Expression 4]

$$I = \min_{i:\ \text{all irradiatuion spots inside layer}} (I_i) \tag{4}$$

In this manner, all of the irradiation spots inside the layer can be irradiated at the ratio F of the movement and stopping or greater. For example, F (stopping time / moving time) = 3 is selected and calculated. In this manner, for all of the irradiation spots inside the layer, it is possible to secure the stopping time which is three times or longer than the moving time. The beam emitted from the accelerator 20 is controlled so as to have constant beam intensity by controlling the accelerator 20. However, if the beam intensity is observed in detail in terms of time, the beam intensity fluctuates at all times with a certain fluctuation width. In the present embodiment, the stopping time is provided with a margin through the above-described algorithm. Therefore, even in a case where the current intensity of the beam increases, it is possible to prevent the irradiation dose from being completely consumed during the moving time.

However, if F increases, a possibility that the irradiation dose may be completely consumed during the moving time decreases, and stable irradiation can be performed in the continuous beam irradiation. In contrast, the beam intensity is lowered, based on Expression (3). That is, beam intensity I for irradiating the layer is also lowered. Since a treatment time T is a value obtained in such a way that all of the irradiation doses are divided by the beam intensity, there is a problem in that the treatment time T is lengthened.

[Expression 5]

$$T = \frac{\sum_{i:\text{all irradiation spots}} MU_i}{I} \tag{5}$$

In particular, in the continuous beam irradiation, in a case where scanning is performed while the beam is turned on between remotely separated irradiation spots, the moving time t is lengthened in Expressions (1) and (2). Therefore, it is necessary to lower the beam intensity, based on Expression (3) .

In Fig. 14, two remotely separated irradiation spots 55 are present. As illustrated in Fig. 13, in a case where the beam moves between the spots while the continuous beam irradiation is performed, it is necessary to lower the beam intensity. Therefore, in Step 108, the planning unit 3 always turns off the beam in a case where the beam moves between the groups after the continuous beam irradiation is performed. In this manner, even if there is a remotely separated spot in a case of the continuous beam irradiation, the continuous beam irradiation can be performed without lowering the beam intensity, and the treatment time can be shortened.

According to the present embodiment, the irradiation spot whose distance from a certain irradiation spot is equal to or greater than a threshold value is classified into another group, and the beam emission is stopped between the groups. Accordingly, even in a case where an important organ is present between the remotely separated irradiation spots, the beam is turned off on the route passing through the important organ. The position of the important organ does not limit how to select the initial irradiation spot (starting point) and the last irradiation spot (end point) inside each group.

Therefore, a shorter scanning route inside each group can be selected, and the beam scanning time can be shortened. Accordingly, the treatment time can be shortened.

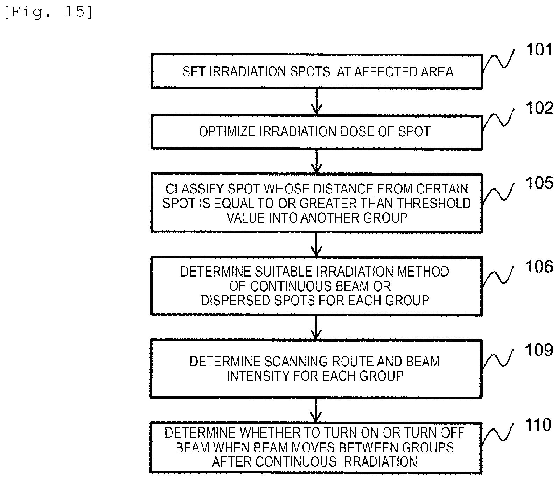

According to the present embodiment, as illustrated in Fig. 9, the scanning route which indicates the beam irradiation order is determined (Step 103). Thereafter, a distance between the adjacent irradiation spots is determined. In a case where the distance is equal to or greater than a threshold value, the irradiation spot is classified into another group, thereby grouping the irradiation spots (Step 104). However, instead of Step 103 and Step 104, as illustrated in Fig. 15, in view of a distance relationship between a plurality of irradiation spots inside the layer, a group classification process (Step 105) may be performed on the irradiation spots. Specifically, the group classification based on the distance relationship means that the irradiation spots inside the layer are classified into the groups by repeating the following process. If the distance between two selected irradiation spots is smaller than the threshold value, the irradiation spots are classified into the same group. If the distance is equal to or greater than the threshold value, the irradiation spots are classified into another group. The irradiation method is selected similarly to Step 106 in Fig. 9. Next, the scanning route and the beam intensity for each group are determined (Step 109), and the starting point and the end point for each group are also determined. In this case, the beam intensity for each group may be changed. Therefore, the distance from the end point of the group before the movement to the starting point of the group after then movement is used so as to determine whether to turn on or off the beam during the movement between the groups after the continuous beam irradiation is performed (Step 110). In a case of Fig. 10, the scanning route for each group can be determined. Therefore, the beam scanning time can be further shortened, and the treatment time can be shortened.

[Embodiment 2]

According to an embodiment of the present invention, the irradiation spots are classified into groups depending on a target irradiation dose.

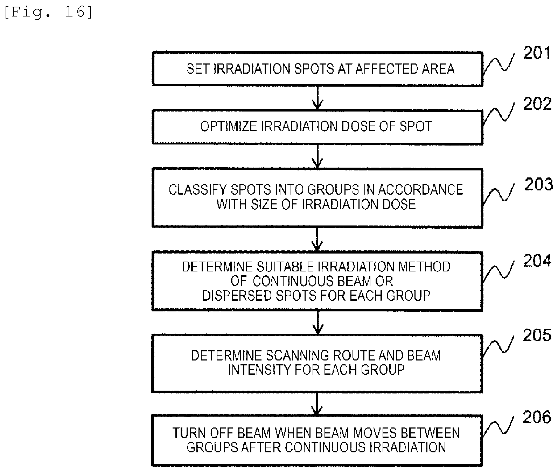

Fig. 16 illustrates a flowchart according to the present embodiment. Similarly to Embodiment 1, the spot determination unit 1 included in the treatment planning device 10 first sets the irradiation spot 53 (Step 201). Thereafter, the irradiation dose is optimized (Step 202), and the target irradiation dose for each spot is determined. These processes correspond to Step 101 and Step 102 in Embodiment 1. Next, the group classification unit 2 classifies the spots into the groups in accordance with a magnitude of the target irradiation dose for each spot (Step 203). For example, in a case where the spots are classified into two groups, a spot having the more target irradiation dose than a certain target irradiation dose is classified into a first group, and a spot having the less target irradiation dose than the certain target irradiation dose is classified into a second group. The number of groups is not limited to two. The spots may be classified into two or more groups.

Next, based on the number of the irradiation spots included in each group, it is determined whether to perform the continuous beam irradiation or the dispersed spot irradiation (Step 204) . With regard to the group on which the continuous beam irradiation is performed in Step 204, the scanning route and the beam intensity are determined (Step 205) similarly to Embodiment 1. The beam intensity is determined as described in Embodiment 1. Step 204 and Step 205 respectively correspond to Step 106 and Step 109 in Embodiment 1. In Step 206, in a case where the continuous beam irradiation is performed on irradiation spots of a certain group, the planning unit 3 causes the beam to move after turning off the beam when the irradiation of the last spot is completed.

As described above, in the second embodiment, the irradiation spots are classified into the groups, based on the magnitude of the target irradiation dose. As a result, in the group having the more target irradiation dose to be given in the continuous beam irradiation, $MU_i$ in Expression (3) increases, thereby enabling the beam intensity to be further raised compared to the related art. In the group having the less target irradiation dose, the beam intensity does not vary compared to the related art. As a result, the beam intensity of the continuous beam irradiation can be further raised compared to the related art. Therefore, the treatment time can be shortened.

In addition, in the scanning irradiation, a uniform irradiation dose distribution is formed by superimposing multiple irradiation dose distributions using thin beams on each other. Accordingly, when the affected area is in respiratory movement, it is an effective way to perform repaint irradiation for irradiating the same spot with the beam multiple times. However, when the repaint irradiation is performed using the continuous beam, the spot having the more irradiation dose and the spot having the less irradiation dose are mixed with each other. Accordingly, if the repaint irradiation is performed using the same beam intensity, the stopping time is shortened at the spot having the less target irradiation dose. If the stopping time cannot be secured, it is the only method to secure the stopping time by lowering the beam intensity. However, if the beam intensity is lowered, the treatment time is lengthened.

According to the present embodiment, the spot having the less target irradiation dose is classified into the dispersed spot, and is separated from the spot on which the continuous beam irradiation is performed. In this manner, it is no longer necessary to lower the beam intensity even when the repaint irradiation is performed using the continuous beam. In this way, the group classification is performed in accordance with the target irradiation dose, thereby selecting a suitable irradiation method of the continuous beam or the dispersed spot. Accordingly, without prolonging the treatment time even when the repaint irradiation is performed, it is possible to form a satisfactory irradiation dose distribution.

In addition, the dispersed spot irradiation or the continuous beam irradiation is selected for each group. In this manner, the repaint irradiation can be more freely performed. Therefore, the irradiation dose distribution can be satisfactorily formed for a moving target, and the treatment time can be further shortened compared to the related art.

[Embodiment 3]

According to a next embodiment, the irradiation spots are classified into the groups in view of both the distance relationship and the magnitude of the target irradiation dose.

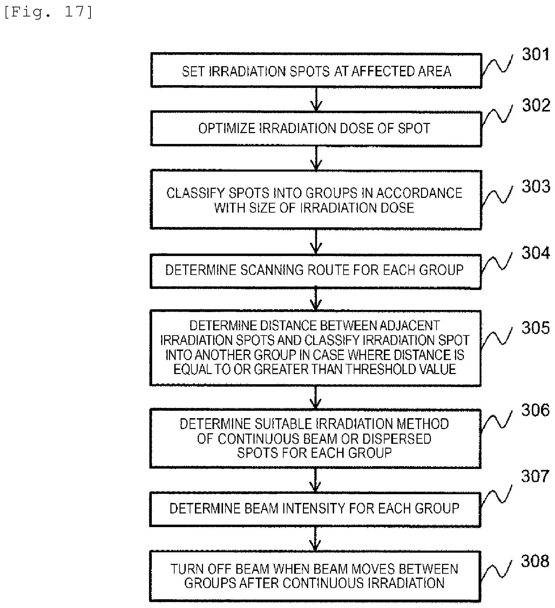

Fig. 17 illustrates a flowchart according to the present embodiment.

Processes until the treatment planning device sets the irradiation spots 53 (Step 301) and the irradiation dose is optimized (Step 302) are the same as those according to Embodiment 1. The processes respectively correspond to Step 101 and Step 102 in Embodiment 1. Next, the group classification unit 2 classifies the spots into the groups in accordance with the magnitude of the target irradiation dose for each spot (Step 303), determines the scanning route for each group (Step 304), calculates the distance between the adjacent irradiation spots, and performs a process for further classifying the irradiation spot separated with a certain distance or farther into another group (Step 305).

If the irradiation spots inside a layer are classified into the plurality of groups, it is determined whether to perform the continuous beam irradiation or the dispersed spot irradiation for each group (Step 306). The beam intensity for each group is determined (Step 307). Similarly to Embodiment 1, the beam is always turned off when the beam moves between the groups after the continuous beam irradiation is performed. The processes respectively correspond to Step 106, Step 107, and Step 108 in Embodiment 1. In Step 307, the beam intensity for each group may be changed.

According to the present embodiment, in the group having the more target irradiation dose to be used for the continuous beam irradiation, the beam intensity can be raised compared to the related art. In addition, even in a case where the important organ is present between the irradiation spots separated from each other with a certain distance or farther inside the group classified in accordance with the target irradiation dose, the beam is turned off on the route passing through the important organ. Accordingly, the position of the important organ does not limit how to select the initial irradiation spot (starting point) and the last irradiation spot (end point) inside each group. Therefore, a shorter scanning route inside each group can be selected, and the beam scanning time can be shortened. Accordingly, the treatment time can be shortened.

In addition, an irradiation method suitable for each group is selected in Step 306. Therefore, the treatment time can be shortened compared to a case where the dispersed spot irradiation is performed on all of the irradiation spots.

In the present embodiment, after the scanning route for each group is determined (Step 304), the distance between the adjacent irradiation spots is calculated, and the process for classifying the irradiation spots separated with a certain distance or farther into another group (Step 305) is performed. However, instead of Step 304 and Step 305, the process for classifying the plurality of irradiation spots inside the layer into the groups may be performed, based on the distance relationship. Specifically, the group classification based on the distance relationship means that the irradiation spots inside the layer are classified into the groups by repeating the following process. If the distance between two selected irradiation spots is smaller than the threshold value, the irradiation spots are classified into the same group, and if the distance is equal to or greater than the threshold value, the irradiation spots are classified into another group.

In addition, in the present embodiment, after the irradiation spots are classified into the groups in accordance with the magnitude of the target irradiation dose, the irradiation spots are classified into the groups in accordance with the distance between the irradiation spots. However, the process order may be reversely performed.

In addition to the synchrotron accelerator 22 described in Embodiments 1 to 3, a cyclotron accelerator may be used.

The invention is defined by the following Claims.

Reference Signs List

| | |
|---|---|
| 1: | SPOT DETERMINATION UNIT |
| 2: | GROUP CLASSIFICATION UNIT |
| 3: | PLANNING UNIT |
| 5: | PATIENT |
| 10: | TREATMENT PLANNING DEVICE |
| 11: | OVERALL CONTROL DEVICE |
| 12: | ACCELERATOR / BEAM TRANSPORT SYSTEM CONTROL DEVICE |
| 13: | IRRADIATION NOZZLE CONTROL DEVICE |

| 20: | ACCELERATOR |
|---|---|
| 21: | BEAM EMITTER |
| 22: | SYNCHROTRON ACCELERATOR |
| 30: | BEAM TRANSPORT SYSTEM |
| 31: | DEFLECTION ELECTROMAGNET |
| 40: | IRRADIATION NOZZLE |
| 41A, 41B: | SCANNING ELECTROMAGNET |
| 42: | IRRADIATION DOSE MONITOR |
| 43: | POSITION MONITOR |
| 44: | RIDGE FILTER |
| 45: | RANGE SHIFTER |
| 50: | TREATMENT TABLE |
| 51: | AFFECTED AREA |
| 52: | LAYER OF AFFECTED AREA TO BE IRRADIATED WITH SAME ENERGY |
| 53: | IRRADIATION SPOT |
| 55: | REMOTELY SEPARATED IRRADIATION SPOT |
| 56-1, 2, 3: | GROUP OF IRRADIATION SPOTS |
| 61A, 61B: | SCANNING ELECTROMAGNET POWER SUPPLY |
| 71: | SCANNING ELECTROMAGNET POWER SUPPLY CONTROL DEVICE |
| 72: | IRRADIATION DOSE MONITOR CONTROL DEVICE |
| 73: | POSITION MONITOR CONTROL DEVICE |
| 81: | BRAGG CURVE |
| 82: | SOBP (SPREAD OUT BRAGG PEAK) |
| 90: | CHARGED PARTICLE BEAM |

**Claims**

1. A treatment planning device comprising:

   a spot determination unit (1) that is configured to classify an irradiation region to be irradiated with a charged particle beam into a plurality of layers (52) in an irradiation direction of the charged particle beam, and that is configured to arrange a plurality of irradiation spots (53) which serve as irradiation positions of the charged particle beam, in the layers (52);
   a group classification unit (2) that is configured to classify the irradiation spots (53) into groups in accordance with a target irradiation dose of each irradiation spot (53); and
   a planning unit (3) that is configured to prepare a treatment plan so as to continuously emit the charged particle beam while the irradiation position is changed from an irradiation spot (53) to a subsequent irradiation spot (53) belonging to a certain group, and so as to stop emitting the charged particle beam while the irradiation position is changed from an irradiation spot (53) belonging to the certain group to an irradiation spot (53) belonging to another group located in the same layer (52) as that of the certain group; and
   wherein the planning unit (3) is configured to determine beam intensity for each of the groups classified by the group classification unit (2).

2. The treatment planning device according to Claim 1,
   wherein the group classification unit (2) is further configured to classify the irradiation spots (53) into groups in accordance with a distance between one irradiation spot (53) and another irradiation spot (53) which are arranged in the same layer (52) such that an irradiation spot (53) whose distance from a certain irradiation spot (53) is equal to or greater than a threshold value is classified into another group.

3. The treatment planning device according to any one of Claims 1 to 2,
   wherein for each group, the group classification unit (2) is configured to determine a sequence of the irradiation spots (53) to be irradiated with the charged particle beam.

4. The treatment planning device according to a Claim 2, wherein the group classification unit (2) is configured to determine sequence of the irradiation spots (53) to be irradiated with the charged particle beam, and to classify a certain irradiation spot (53) into another group if a distance between the certain irradiation spot (53) and the irradiation spot (53) to be subsequently irradiated is equal to or greater than the threshold value.

**5.** The treatment planning device according to Claim 1,
wherein the group classification unit (2) is configured to classify the irradiation spots (53) whose target irradiation dose falls within a predetermined range, into the same group.

**6.** A computer-implemented treatment planning method comprising:

a first step of classifying an irradiation region to be irradiated with a charged particle beam into a plurality of layers (52) in an irradiation direction of the charged particle beam, and of performing spot arrangement for arranging a plurality of irradiation spots (53) which serve as irradiation positions of the charged particle beam, in the layers (52);
a second step of classifying the irradiation spots (53) into groups in accordance with a target irradiation dose of each irradiation spot (53) and determining the beam intensities for each of the groups classified by the group classification; and
a third step of planning to output a signal to start to emit the charged particle beam while the irradiation position is changed from the irradiation spot (53) to the subsequent irradiation spot (53) belonging to a certain group, and to output a signal to stop emitting the charged particle beam while the irradiation position is changed from the irradiation spot (53) belonging to a certain group to the irradiation spot(53) belonging to another group located in the same layer (52) as that of the certain group.

**7.** A control device that controls a charged particle beam to be emitted to a plurality of irradiation spots (53) arranged in a plurality of layers (52) into which an irradiation region to be irradiated with a charged particle beam is classified in an irradiation direction of the charged particle beam,
wherein the control device is configured to output a continuous beam emission signal so as to continuously emit the charged particle beam to the irradiation spots classified into groups in accordance with a target irradiation dose of each irradiation spot (53), while an irradiation position is changed from an irradiation spot (53) belonging to a certain group to a subsequent irradiation spot (53) located in the same layer (52), wherein the beam intensities are determined for each of the groups classified by the group classification, and
wherein the control device is configured to output a beam stop signal so as to stop emitting the charged particle beam, while the irradiation position is changed from an irradiation spot (53) belonging to a certain group to an irradiation spot (53) belonging to another group located in the same layer (52) as that of the certain group.

**8.** A particle beam treatment device comprising:

an acceleration device that is configured to accelerate a charged particle beam;
an irradiation device that is configured to emit the charged particle beam to a plurality of irradiation spots (53) arranged in layers (52) into which an irradiation region to be irradiated with the charged particle beam is classified in an irradiation direction of the charged particle beam; and
a control device that is configured to control the acceleration device and the irradiation device,
wherein the control device is configured to output a continuous beam emission signal so as to continuously emit the charged particle beam to the irradiation spots (53) classified into groups in accordance with a target irradiation dose of each irradiation spot (53), while an irradiation position is changed from an irradiation spot (53) belonging to a certain group to a subsequent irradiation spot (53) belonging to the certain group, wherein the beam intensities are determined for each of the groups classified by the group classification, and
wherein the control device is configured to output a beam stop signal so as to stop emitting the charged particle beam, while the irradiation position (53) is changed from an irradiation spot (53) belonging to a certain group to an irradiation spot (53) belonging to another group located in the same layer (52) as that of the certain group.

**Patentansprüche**

**1.** Behandlungsplanungsvorrichtung, die Folgendes umfasst:

eine Fleckbestimmungseinheit (1), die konfiguriert ist, einen Bestrahlungsbereich, der mit einem Ladungsträgerstrahl zu bestrahlen ist, in einer Bestrahlungsrichtung des Ladungsträgerstrahls in mehrere Schichten (52) einzuordnen, und die konfiguriert ist, mehrere Bestrahlungsflecken (53), die als Bestrahlungspositionen des Ladungsträgerstrahls dienen, in den Schichten (52) anzuordnen;
eine Gruppeneinordnungseinheit (2), die konfiguriert ist, die Bestrahlungsflecken (53) in Übereinstimmung mit einer Zielbestrahlungsdosierungjedes Bestrahlungsflecks (53) in Gruppen einzuordnen; und

eine Planungseinheit (3), die konfiguriert ist, einen Behandlungsplan zu erstellen, um den Ladungsträgerstrahl kontinuierlich auszusenden, während die Bestrahlungsposition von einem Bestrahlungsfleck (53) zu einem folgenden Bestrahlungsfleck (53), der zu einer bestimmen Gruppe gehört, geändert wird, und um das Aussenden des Ladungsträgerstrahls zu beenden, während die Bestrahlungsposition von einem Bestrahlungsfleck (53), der zu der bestimmten Gruppe gehört, zu einem Bestrahlungsfleck (53), der zu einer anderen Gruppe gehört, die sich in derselben Schicht (52) wie jene der bestimmten Gruppe befindet, geändert wird; und wobei die Planungseinheit (3) konfiguriert ist, eine Strahlstärke für jede der durch die Gruppeneinordnungseinheit (2) eingeordneten Gruppen zu bestimmen.

2. Behandlungsplanungsvorrichtung nach Anspruch 1,
wobei die Gruppeneinordnungseinheit (2) ferner konfiguriert ist, die Bestrahlungsflecken (53) in Übereinstimmung mit einem Abstand zwischen einem Bestrahlungsfleck (53) und einem anderen Bestrahlungsfleck (53), die in derselben Schicht (52) angeordnet sind, in Gruppen einzuordnen, so dass ein Bestrahlungsfleck (53), dessen Abstand von einem bestimmen Bestrahlungsfleck (53) gleich oder größer als ein Schwellenwert ist, in eine andere Gruppe eingeordnet wird.

3. Behandlungsplanungsvorrichtung nach einem der Ansprüche 1 bis 2,
wobei für jede Gruppe die Gruppeneinordnungseinheit (2) konfiguriert ist, eine Folge der Bestrahlungsflecken (53), die mit dem Ladungsträgerstrahl zu bestrahlen sind, zu bestimmen.

4. Behandlungsplanungsvorrichtung nach Anspruch 2,
wobei die Gruppeneinordnungseinheit (2) konfiguriert ist, eine Folge der Bestrahlungsflecken (53), die mit dem Ladungsträgerstrahl zu bestrahlen sind, zu bestimmen und einen bestimmten Bestrahlungsfleck (53) in eine andere Gruppe einzuordnen, wenn ein Abstand zwischen dem bestimmten Bestrahlungsfleck (53) und dem Bestrahlungsfleck (53), der anschließend zu bestrahlen ist, gleich dem Schwellenwert oder größer als dieser ist.

5. Behandlungsplanungsvorrichtung nach Anspruch 1,
wobei die Gruppeneinordnungseinheit (2) konfiguriert ist, die Bestrahlungsflecken (53), deren Zielbestrahlungsdosierung in einen vorbestimmten Bereich fällt, in dieselbe Gruppe einzuordnen.

6. Computerimplementiertes Behandlungsplanungsverfahren, das Folgendes umfasst:

einen ersten Schritt des Einordnens eines Bestrahlungsbereichs, der mit einem Ladungsträgerstrahl zu bestrahlen ist, in einer Bestrahlungsrichtung des Ladungsträgerstrahls in mehrere Schichten (52) und des Ausführens einer Fleckenanordnung zum Anordnen mehrerer Bestrahlungsflecken (53), die als Bestrahlungspositionen des Ladungsträgerstrahls dienen, in den Schichten (52);
einen zweiten Schritt des Einordnens der Bestrahlungsflecken (53) in Übereinstimmung mit einer Zielbestrahlungsdosierung jedes Bestrahlungsflecks (53) in Gruppen und des Bestimmens der Strahlstärken für jede der durch die Gruppeneinordnung eingeordneten Gruppen; und
einen dritten Schritt des Planens, ein Signal auszugeben, um mit dem Aussenden des Ladungsträgerstrahls zu beginnen, während die Bestrahlungsposition von dem Bestrahlungsfleck (53) zu dem folgenden Bestrahlungsfleck (53), der zu einer bestimmten Gruppe gehört, geändert wird, und ein Signal auszugeben, um das Aussenden des Ladungsträgerstrahls anzuhalten, während die Bestrahlungsposition von dem Bestrahlungsfleck (53), der zu einer bestimmten Gruppe gehört, zu dem Bestrahlungsfleck (53), der zu einer anderen Gruppe gehört, die sich in derselben Schicht (52) wie jene der bestimmten Gruppe befindet, geändert wird.

7. Steuervorrichtung, die einen Ladungsträgerstrahl steuert, der an mehrere Bestrahlungsflecken (53) auszusenden ist, die in mehreren Schichten (52) angeordnet sind, in die ein Bestrahlungsbereich, der mit einem Ladungsträgerstrahl zu bestrahlen ist, in einer Bestrahlungsrichtung des Ladungsträgerstrahls eingeordnet ist,
wobei die Steuervorrichtung konfiguriert ist, ein kontinuierliches Strahlaussendungssignal auszugeben, um den Ladungsträgerstrahl an die Bestrahlungsflecken kontinuierlich auszusenden, die in Übereinstimmung mit einer Zielbestrahlungsdosierung jedes Bestrahlungsflecks (53) in Gruppen eingeordnet sind, während eine Bestrahlungsposition von einem Bestrahlungsfleck (53), der zu einer bestimmten Gruppe gehört, zu einem folgenden Bestrahlungsfleck (53), der sich in derselben Schicht (52) befindet, geändert wird, wobei die Strahlstärken für jede der durch die Gruppeneinordnung eingeordneten Gruppen bestimmt werden, und
wobei die Steuervorrichtung konfiguriert ist, ein Strahlbeendigungssignal auszugeben, um das Aussenden des Ladungsträgerstrahls zu beenden, während die Bestrahlungsposition von einem Bestrahlungsfleck (53), der zu einer bestimmten Gruppe gehört, zu einem Bestrahlungsfleck (53), der zu einer anderen Gruppe gehört, die sich in derselben Schicht (52) wie jene der bestimmten Gruppe befindet, geändert wird.

8.  Ladungsträgerstrahl-Behandlungsvorrichtung, die Folgendes umfasst:

    eine Beschleunigungsvorrichtung, die konfiguriert ist, einen Ladungsträgerstrahl zu beschleunigen;
    eine Bestrahlungsvorrichtung, die konfiguriert ist, den Ladungsträgerstrahl zu mehreren Bestrahlungsflecken (53), die in Schichten (52) angeordnet sind, in die ein Bestrahlungsbereich, der mit dem Ladungsträgerstrahl zu bestrahlen ist, in einer Bestrahlungsrichtung des Ladungsträgerstrahls eingeordnet ist, auszusenden; und
    eine Steuervorrichtung, die konfiguriert ist, die Beschleunigungsvorrichtung und die Bestrahlungsvorrichtung zu steuern,
    wobei die Steuervorrichtung konfiguriert ist, ein kontinuierliches Strahlaussendungssignal auszugeben, um den Ladungsträgerstrahl zu den Bestrahlungsflecken (53), die in Übereinstimmung mit einer Zielbestrahlungsdosierungjedes Bestrahlungsflecks (53) in Gruppen eingeordnet sind, kontinuierlich auszusenden, während eine Bestrahlungsposition von einem Bestrahlungsfleck (53), der zu einer bestimmten Gruppe gehört, zu einem folgenden Bestrahlungsfleck (53), der zu der bestimmten Gruppe gehört, geändert wird, wobei die Strahlstärken für jede der durch die Gruppeneinordnung eingeordneten Gruppen bestimmt werden, und
    wobei die Steuervorrichtung konfiguriert ist, ein Strahlbeendigungssignal auszugeben, um das Aussenden des Ladungsträgerstrahl zu beenden, während die Bestrahlungsposition (53) von einem Bestrahlungsfleck (53), der zu einer bestimmten Gruppe gehört, zu einem Bestrahlungsfleck (53), der zu einer anderen Gruppe gehört, die sich in derselben Schicht (52) wie jene der bestimmten Gruppe befindet, geändert wird.

## Revendications

1.  Dispositif de planification de traitement, comprenant :

    une unité de détermination de point (1) qui est configurée pour classifier une région d'irradiation qu'il s'agit d'irradier avec un faisceau de particules chargées dans une pluralité de couches (52) dans une direction d'irradiation du faisceau de particules chargées, et qui est configurée pour agencer une pluralité de points d'irradiation (53), qui servent de positions d'irradiation du faisceau de particules chargées, dans les couches (52) ;
    une unité de classification de groupes (2) qui est configurée pour classifier les points d'irradiation (53) dans des groupes en accord avec une dose d'irradiation cible de chaque point d'irradiation (53) ; et
    une unité de planification (3) qui est configurée pour préparer un plan de traitement de manière à émettre en continu le réseau de particules chargées tandis que la position d'irradiation est changée depuis un point d'irradiation (53) à un point d'irradiation (53) suivant appartenant à un certain groupe, et de manière à arrêter d'émettre le faisceau de particules chargées tandis que la position d'irradiation est changée depuis un point d'irradiation (53) appartenant à un certain groupe jusqu'à un point d'irradiation (53) appartenant à un autre groupe situé dans la même couche (52) que celle du certain groupe ; et
    dans lequel l'unité de planification (3) est configurée pour déterminer une intensité de faisceau pour chacun des groupes classifiés par l'unité de classification de groupes (2).

2.  Dispositif de planification de traitement selon la revendication 1, dans lequel l'unité de classification de groupes (2) est en outre configurée pour classifier les points d'irradiation (53) dans des groupes en accord avec une distance entre un point d'irradiation (53) et un autre point d'irradiation (53) qui sont agencés dans la même couche (52) de sorte qu'un point d'irradiation (53), dont la distance par rapport à un certain point d'irradiation (53) est égale ou supérieure à une valeur seuil, est classifié dans un autre groupe.

3.  Dispositif de planification de traitement selon l'une quelconque des revendications 1 à 2,
    dans lequel, pour chaque groupe, l'unité de classification de groupes (2) est configurée pour déterminer une séquence des points d'irradiation (53) qu'il s'agit d'irradier avec le faisceau de particules chargées.

4.  Dispositif de planification de traitement selon la revendication 2, dans lequel l'unité de classification de groupes (2) est configurée pour déterminer une séquence des points d'irradiation (53) qu'il s'agit d'irradier avec le faisceau de particules chargées, et pour classifier un certain point d'irradiation (53) dans un autre groupe si une distance entre le certain point d'irradiation (53) et le point d'irradiation (53) qu'il s'agit d'irradier par la suite est égale ou supérieure à la valeur seuil.

5.  Dispositif de planification de traitement selon la revendication 1, dans lequel l'unité de classification de groupes (2) est configurée pour classifier les points d'irradiation (53), dont la dose d'irradiation cible tombe à l'intérieur d'une plage prédéterminée, dans le même groupe.

6. Procédé de planification de traitement mis en œuvre dans un ordinateur, comprenant :

une première étape consistant à classifier une région d'irradiation qu'il s'agit d'irradier avec un faisceau de particules chargées dans une pluralité de couches (52) dans une direction d'irradiation du faisceau de particules chargées, et à exécuter un agencement de points pour agencer une pluralité de points d'irradiation (53), qui servent de position d'irradiation du faisceau de particules chargées, dans les couches(52);

une seconde étape consistant à classifier les points d'irradiation (53) dans des groupes en accord avec une dose d'irradiation cible de chaque point d'irradiation (53) et à déterminer les intensités de faisceau pour chacun des groupes classifiés par la classification de groupes ; et

une troisième étape de planification pour sortir un signal pour commencer à émettre le réseau de particules chargées tandis que la position d'irradiation est changée depuis le point d'irradiation (53) jusqu'au point d'irradiation (53) suivant appartenant à un certain groupe, et pour sortir un signal pour arrêter d'émettre le faisceau de particules chargées tandis que la position d'irradiation est changée depuis le point d'irradiation (53) appartenant à un certain groupe jusqu'au point d'irradiation (53) appartenant à un autre groupe situé dans la même couche (52) que celle du certain groupe.

7. Dispositif de commande qui commande un faisceau de particules chargées qu'il s'agit d'émettre jusqu'à une pluralité de points d'irradiation (53) agencés dans une pluralité de couches (52) dans lesquelles une région d'irradiation qu'il s'agit d'irradier avec un faisceau de particules chargées est classifiée dans une direction d'irradiation du faisceau de particules chargées,

dans lequel le dispositif de commande est configuré pour sortir un signal d'émission de faisceau continu de manière à émettre en continu le faisceau de particules chargées jusqu'aux points d'irradiation classifiés dans des groupes en accord avec une dose d'irradiation cible de chaque point d'irradiation (53) tandis qu'une position d'irradiation est changée depuis un point d'irradiation (53) appartenant à un certain groupe jusqu'à un point d'irradiation (53) suivant situé dans la même couche (52),

dans lequel les intensités de faisceau sont déterminées pour chacun des groupes classifiés par la classification de groupes, et

dans lequel le dispositif de commande est configuré pour sortir un signal d'arrêt de faisceau de manière à arrêter d'émettre le faisceau de particules chargées tandis que la position d'irradiation est changée depuis un point d'irradiation (53) appartenant à un certain groupe jusqu'à un point d'irradiation (53) appartenant à un autre groupe situé dans la même couche (52) que celle du certain groupe.

8. Dispositif de traitement par faisceau de particules comprenant :

un dispositif d'accélération qui est configuré pour accélérer un faisceau de particules chargées ;

un dispositif d'irradiation qui est configuré pour émettre le faisceau de particules chargées jusqu'à une pluralité de points d'irradiation (53) agencés en couches (52) dans lesquelles une région d'irradiation qu'il s'agit d'irradier avec le faisceau de particules chargées est classifiée dans une direction d'irradiation du faisceau de particules chargées ; et

un dispositif de commande qui est configuré pour commander le dispositif d'accélération et le dispositif d'irradiation,

dans lequel le dispositif de commande est configuré pour sortir un signal d'émission de faisceau continu de manière à émettre en continu le faisceau de particules chargées jusqu'aux points d'irradiation (53) classifiés dans des groupes en accord avec une dose d'irradiation cible de chaque point d'irradiation (53) tandis qu'une position d'irradiation est changée depuis un point d'irradiation (53) appartenant à un certain groupe jusqu'à un point d'irradiation (53) suivant appartenant au certain groupe, dans lequel les intensités de faisceau sont déterminées pour chacun des groupes classifiés par la classification de groupe, et

dans lequel le dispositif de commande est configuré pour sortir un signal d'arrêt de faisceau de manière à arrêter d'émettre le faisceau de particules chargées tandis que la position d'irradiation (53) est changée depuis un point d'irradiation (53) appartenant à un certain groupe jusqu'à un point d'irradiation (53) appartenant à un autre groupe situé dans la même couche (52) que celle du certain groupe.

[Fig. 1]

[Fig. 2]

[Fig. 3]

53

90

51

52

[Fig. 4]

82

IRRADIATION DOSE

81

DEPTH

[Fig. 5]

[Fig. 6]

[Fig. 7]

| SPOT IRRADIATION SEQUENCE | SPOT 1 | SPOT 2 | SPOT 3 |

BEAM INTENSITY

TIME

IRRADIATION DOSE MONITOR COUNTING

COMPLETION    COMPLETION    COMPLETION

TIME

SCANNING ELECTROMAGNET CURRENT

STOPPING   MOVING   STOPPING   MOVING   STOPPING

TIME

POSITION MONITOR CALCULATION

POSITION / WIDTH CALCULATION    POSITION / WIDTH CALCULATION    POSITION / WIDTH CALCULATION

TIME

[Fig. 8]

[Fig. 9]

```
┌─────────────────────────────────────────┐
│    SET IRRADIATION SPOTS AT AFFECTED AREA │ ⟿ 101
└─────────────────────────────────────────┘
                    ↓
┌─────────────────────────────────────────┐
│      OPTIMIZE IRRADIATION DOSE OF SPOT    │ ⟿ 102
└─────────────────────────────────────────┘
                    ↓
┌─────────────────────────────────────────┐
│         DETERMINE SCANNING ROUTE          │ ⟿ 103
└─────────────────────────────────────────┘
                    ↓
┌─────────────────────────────────────────┐
│   DETERMINE DISTANCE BETWEEN ADJACENT     │
│ IRRADIATION SPOTS AND CLASSIFY IRRADIATION SPOT │ ⟿ 104
│  INTO ANOTHER GROUP IN CASE WHERE DISTANCE IS │
│    EQUAL TO OR GREATER THAN THRESHOLD VALUE │
└─────────────────────────────────────────┘
                    ↓
┌─────────────────────────────────────────┐
│  DETERMINE SUITABLE IRRADIATION METHOD    │
│   OF CONTINUOUS BEAM OR DISPERSED         │ ⟿ 106
│         SPOTS FOR EACH GROUP              │
└─────────────────────────────────────────┘
                    ↓
┌─────────────────────────────────────────┐
│   DETERMINE BEAM INTENSITY FOR EACH GROUP │ ⟿ 107
└─────────────────────────────────────────┘
                    ↓
┌─────────────────────────────────────────┐
│  TURN OFF BEAM WHEN BEAM MOVES BETWEEN    │
│   GROUPS AFTER CONTINUOUS IRRADIATION     │ ⟿ 108
└─────────────────────────────────────────┘
```

[Fig. 10]

EP 3 199 202 B1

[Fig. 11]

56-1

56-2

56-3

Y

X

[Fig. 12]

56-1

56-2

56-3

Y

X

22

[Fig. 13]

IRRADIATION SPOT i
IRRADIATION DOSE MU$_i$

STOPPING TIME FXT
F×t

MOVING TIME t

IRRADIATION SPOT (i-1)

[Fig. 14]

[Fig. 15]

```
┌────────────────────────────────────────┐
│   SET IRRADIATION SPOTS AT AFFECTED AREA │ ⟿ 101
└────────────────────────────────────────┘
                    │
                    ▼
┌────────────────────────────────────────┐
│     OPTIMIZE IRRADIATION DOSE OF SPOT    │ ⟿ 102
└────────────────────────────────────────┘
                    │
                    ▼
┌────────────────────────────────────────┐
│  CLASSIFY SPOT WHOSE DISTANCE FROM CERTAIN│
│  SPOT IS EQUAL TO OR GREATER THAN THRESHOLD│ ⟿ 105
│         VALUE INTO ANOTHER GROUP          │
└────────────────────────────────────────┘
                    │
                    ▼
┌────────────────────────────────────────┐
│   DETERMINE SUITABLE IRRADIATION METHOD  │
│          OF CONTINUOUS BEAM OR           │ ⟿ 106
│      DISPERSED SPOTS FOR EACH GROUP      │
└────────────────────────────────────────┘
                    │
                    ▼
┌────────────────────────────────────────┐
│    DETERMINE SCANNING ROUTE AND BEAM     │
│         INTENSITY FOR EACH GROUP         │ ⟿ 109
└────────────────────────────────────────┘
                    │
                    ▼
┌────────────────────────────────────────┐
│ DETERMINE WHETHER TO TURN ON OR TURN OFF │
│  BEAM WHEN BEAM MOVES BETWEEN GROUPS     │ ⟿ 110
│       AFTER CONTINUOUS IRRADIATION       │
└────────────────────────────────────────┘
```

[Fig. 16]

```
┌─────────────────────────────────────────┐
│   SET IRRADIATION SPOTS AT AFFECTED AREA │╲╱ 201
└─────────────────────────────────────────┘
                    │
                    ▼
┌─────────────────────────────────────────┐
│     OPTIMIZE IRRADIATION DOSE OF SPOT    │╲╱ 202
└─────────────────────────────────────────┘
                    │
                    ▼
┌─────────────────────────────────────────┐
│   CLASSIFY SPOTS INTO GROUPS IN ACCORDANCE│╲╱ 203
│      WITH SIZE OF IRRADIATION DOSE       │
└─────────────────────────────────────────┘
                    │
                    ▼
┌─────────────────────────────────────────┐
│   DETERMINE SUITABLE IRRADIATION METHOD  │╲╱ 204
│         OF CONTINUOUS BEAM OR            │
│      DISPERSED SPOTS FOR EACH GROUP      │
└─────────────────────────────────────────┘
                    │
                    ▼
┌─────────────────────────────────────────┐
│      DETERMINE SCANNING ROUTE AND BEAM   │╲╱ 205
│           INTENSITY FOR EACH GROUP       │
└─────────────────────────────────────────┘
                    │
                    ▼
┌─────────────────────────────────────────┐
│  TURN OFF BEAM WHEN BEAM MOVES BETWEEN   │╲╱ 206
│    GROUPS AFTER CONTINUOUS IRRADIATION   │
└─────────────────────────────────────────┘
```

[Fig. 17]

```
┌─────────────────────────────────────────┐
│   SET IRRADIATION SPOTS AT AFFECTED AREA  │ ～ 301
└─────────────────────────────────────────┘
                    ↓
┌─────────────────────────────────────────┐
│      OPTIMIZE IRRADIATION DOSE OF SPOT     │ ～ 302
└─────────────────────────────────────────┘
                    ↓
┌─────────────────────────────────────────┐
│  CLASSIFY SPOTS INTO GROUPS IN ACCORDANCE  │ ～ 303
│      WITH SIZE OF IRRADIATION DOSE         │
└─────────────────────────────────────────┘
                    ↓
┌─────────────────────────────────────────┐
│   DETERMINE SCANNING ROUTE FOR EACH GROUP  │ ～ 304
└─────────────────────────────────────────┘
                    ↓
┌─────────────────────────────────────────┐
│      DETERMINE DISTANCE BETWEEN ADJACENT    │ ～ 305
│ IRRADIATION SPOTS AND CLASSIFY IRRADIATION SPOT│
│  INTO ANOTHER GROUP IN CASE WHERE DISTANCE IS  │
│  EQUAL TO OR GREATER THAN THRESHOLD VALUE  │
└─────────────────────────────────────────┘
                    ↓
┌─────────────────────────────────────────┐
│     DETERMINE SUITABLE IRRADIATION METHOD   │ ～ 306
│      OF CONTINUOUS BEAM OR DISPERSED        │
│            SPOTS FOR EACH GROUP            │
└─────────────────────────────────────────┘
                    ↓
┌─────────────────────────────────────────┐
│    DETERMINE BEAM INTENSITY FOR EACH GROUP  │ ～ 307
└─────────────────────────────────────────┘
                    ↓
┌─────────────────────────────────────────┐
│   TURN OFF BEAM WHEN BEAM MOVES BETWEEN    │ ～ 308
│    GROUPS AFTER CONTINUOUS IRRADIATION     │
└─────────────────────────────────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5791793 B
- EP 2392383 A1
- US 20150080631 A1
- WO 2014072213 A